# EUROPEAN PATENT SPECIFICATION

(11) **EP 1 502 559 B1**
(45) Date of publication and mention of the grant of the patent: **15.11.2006**
(21) Application number: 04018100.0
(22) Date of filing: 30.07.2004
(51) Int. Cl.: A61F 2/16

(54) **Intraocular lens injector**
Injektionsvorrichtung für eine intra-okulare Linse
Dispositif d'injection de lentille intra-oculaire

(30) Priority: 31.07.2003 JP 2003283564
(43) Date of publication of application: 02.02.2005
(73) Proprietor: Nidek Co., Ltd., Gamagori-shi, Aichi 443-0035 (JP)
(72) Inventor: Sunada, Tsutomu, Toyohashi-shi Aichi-ken 441-3101 (JP); Oda, Haruo, Gamagori-shi Aichi-ken 443-0011 (JP)
(74) Representative: Hofer, Dorothea

(56) References cited:
- WO-A-97/15253
- US-A- 5 620 450
- US-A1- 2001 001 822
- US-A1- 2002 077 633

## Description

### BACKGROUND OF THE INVENTION

### 1. Field of the Invention

The present invention relates to an intraocular lens injector for injecting an intraocular lens into an eye of a patient.

### 2. Description of Related Art

As one of operative treatments for cataract, generally used is a method of removing a crystalline lens from an eye of a patient and then injecting an intraocular lens in place of the crystalline lens. To inject the intraocular lens, the following steps are taken: first making an incision in an eyeball of a patient's eye; fragmenting and aspirating a clouded crystalline lens through the incision by for example an ultrasonic cataract-surgery device (a phaco-emulsification device); and then injecting the intraocular lens into the eye through the incision to implant it in place of the crystalline lens.

If a large incision is made, it may become a burden on the eyeball and also cause astigmatism of the patient's eye after the operation. To prevent such the disadvantages, an intraocular lens injector is used to inject a foldable intraocular lens into an eye through a smaller incision. In this injector, the foldable intraocular lens held in a housing of the injector is pushed toward the tip of the injector while being folded into a smaller shape. Thereafter, the folded intraocular lens is pushed out of the tip of the injector inserted in the eye through the incision and is spread (unfolded) and placed in the eye.

As the above injector, there is a type of an injector constructed such that an intraocular lens is placed in a lens holding unit (i.e., a lens cartridge) and thereafter the lens holding unit is mounted in an injector main unit. Some injectors of this type are adapted to place an intraocular lens in a folded state in the injector, that is, to mount the lens holding unit in the injector main unit after the lens is placed in the folded state in the lens holding unit (see EP 1415619A2 (Japanese patent unexamined publication No. 2004-113610) and Japanese patent unexamined publication No. Hei 05-103803).

US5620450 discloses a deformable intraocular lens injecting apparatus including a transverse hinge for loading a deformable intraocular lens therein. The transverse hinge connects together two portions of the lens injecting apparatus. A preferred embodiment includes a lens injecting device, a lens cartridge having a transverse hinge, and a nozzle portion. In a most preferred embodiment, the lens cartridge includes a fixed lower tray portion transversely hinged to an upper lid portion.

WO9715253 discloses a deformable intraocular lens injecting apparatus having a lens cartridge including a pair of extensions for opening and closing the lens cartridge. In operation, the lens cartridge is loaded with a deformable intraocular lens and a viscoelastic lubricant. The lens cartridge is then loaded into a lens cartridge receiver of the lens injecting device. A further embodiment comprises three separate components including a lens injecting device containing a slidable plunger and a lens cartridge receiver, a transverse hinged lens cartridge having an upper portion and a lower portion connected together by a transverse hinge, and a nozzle portion.

However, manipulations of the above conventional injectors would be troublesome in mounting the lens holding unit in the injector main unit after the lens is placed in the lens holding unit.

### SUMMARY OF THE INVENTION

The present invention has been made in view of the above circumstances and has an object to overcome the above problems and to provide an intraocular lens injector adapted to allow easy folding and placing of an intraocular lens in the injector.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

Such objects are achieved in accordance with an intraocular lens injector comprising the features of claim 1.

Further developments of the present invention are given in the dependent claims.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.

In the drawings,
Figs. 1A and 1B are schematic structural views of an intraocular lens injector in a first embodiment of the present invention;
Fig. 2 is a schematic structural view of an intraocular lens;
Figs. 3A and 3B are schematic structural views of a lens holding unit;
Figs. 4A and 4B are schematic structural views of a lens holding unit;
Fig. 5 is a schematic structural view of a main unit and a push unit;
Fig. 6 is a schematic structural view of a main unit and a push unit; and
Figs. 7A and 7B are schematic structural views of an intraocular lens injector in a second embodiment of the present invention.

### DETAILED DESCRIPTION OF THE PREFERRED EMBODIMENTS

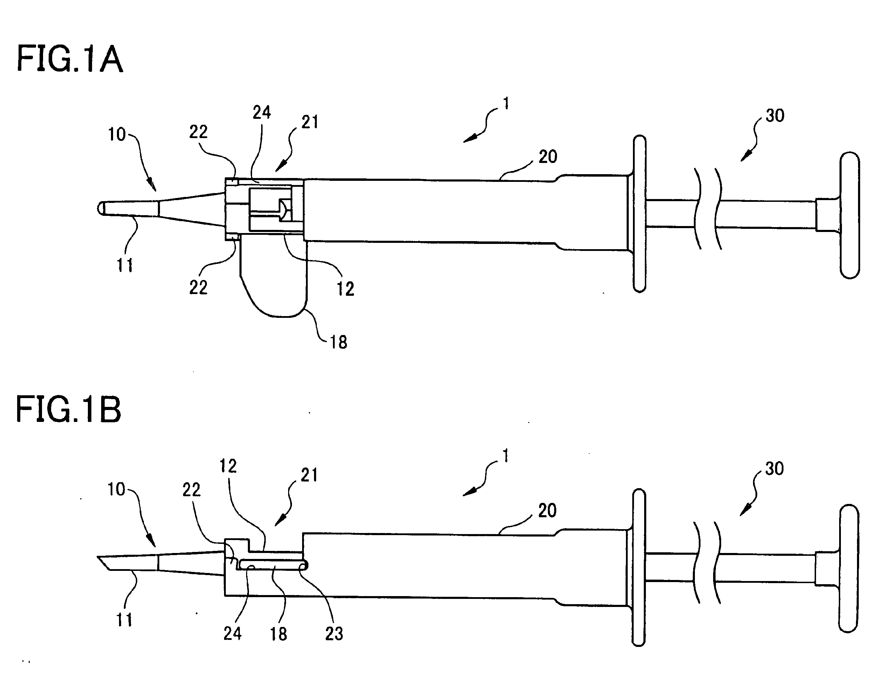

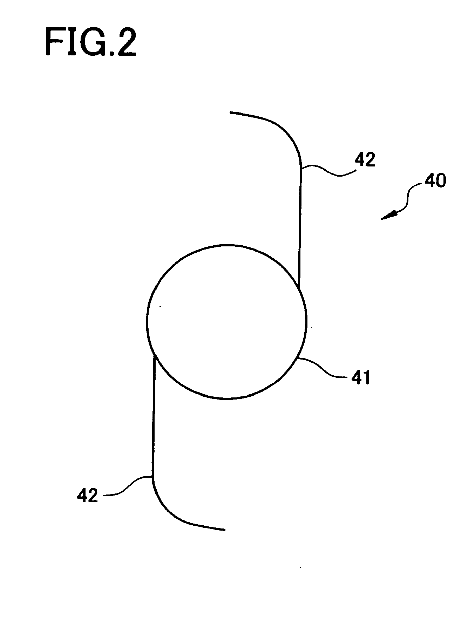

A detailed description of a first embodiment of an intraocular lens injector embodying the present invention will now be given referring to the accompanying drawings. Figs. 1A and 1B are schematic structural views of an intraocular lens injector 1 in the present embodiment; Fig. 1A is a plane view and Fig. 1B is a side view. The injector 1 includes, in order of insertion into an eye, a lens holding unit 10 (a lens cartridge) provided with a cylindrical (tubular) insertion part 11 which is inserted in an incision made in an eyeball and a setting part 12 in which an intraocular lens (hereinafter, IOL) 40 (see Fig. 2) is placed, a cylindrical (tubular) main unit 20 in which the lens holding unit 10 is mounted, and a push unit 30 inserted in the main unit 20 and the lens holding unit 10. This push unit 30 is used to push out the IOL 40 placed in the setting part 12 of the lens holding unit 10 through a front end (a tip end) of the insertion part 11 to the outside.

Fig. 2 is a schematic structural view of the IOL 40. This IOL 40 includes an optical portion 41 having a predetermined refractive power and a pair of support portions 42 which support the optical portion 41 in an eye. The IOL 40 in the present embodiment is made of a material commonly used for an optical portion of a foldable soft intraocular lens, for example, a monomeric material such as hydroxyethyl methacrylate (HEMA) or a composite material such as acrylic ester and methacrylate ester. The support parts 42 are made of a material commonly used for a support portion of an intraocular lens such as polymethyl methacrylate (PMMA).

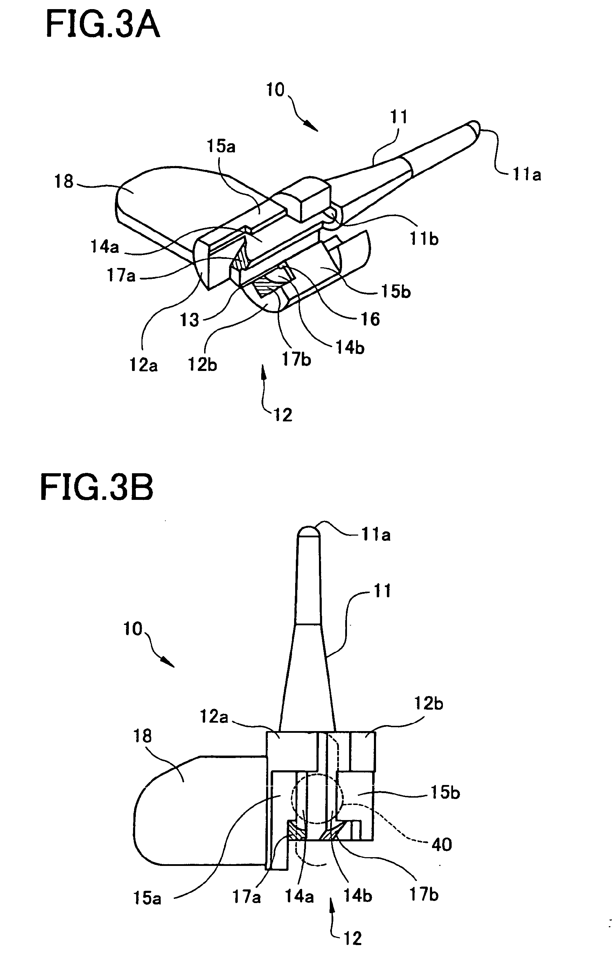

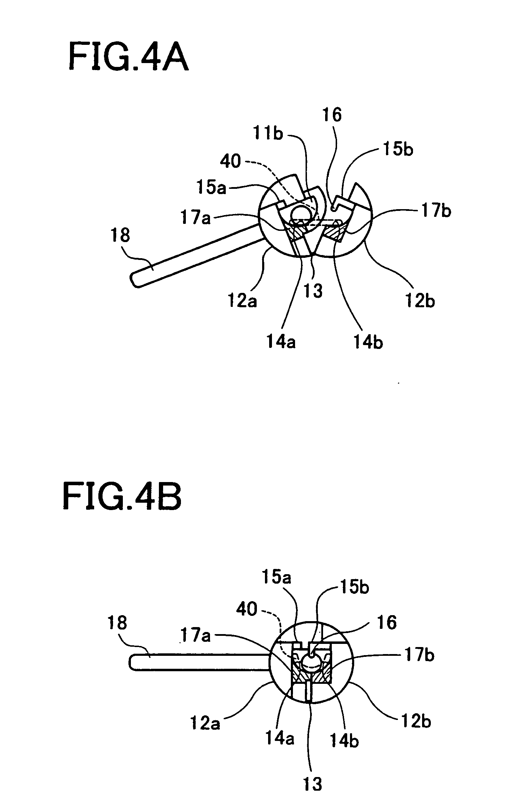

Figs. 3A and 3B and 4A and 4B are schematic structural views of the lens holding unit 10. Specifically, Fig. 3A is a perspective view of the unit 10 seen from behind (opposite the tip end), Fig. 3B is a plane view. Figs. 4A and 4B are back views of the unit 10; Fig. 4A shows an opened state and Fig. 4B shows a closed state. The lens holding unit 10 is entirely made of resin and is a single-use type which is thrown away after one use.

The insertion part 11 of the lens holding unit 10 is formed with a diametrical taper so that its diameter becomes smaller (narrower) toward a tip end 11a which is inserted in an incision made in an eyeball. With this structure, the folded IOL 40 is further folded into a smaller shape while passing through the hollow of a cylindrical (tubular) portion of the insertion part 11 and then the IOL 40 is pushed out through the tip end 11a to the outside.

In the lens holding unit 10, the setting part 12 is arranged on a side opposite the tip end 11a of the insertion part 11 and adapted to be opened and closed about a hinge 13. The setting part 12 includes a fixed part 12a fixed to the insertion part 11 and a movable part (an openable and closable part) 12b connected with the fixed part 12a with the hinge 13. The fixed part 12a has a setting surface 14a and the movable part 12b has a setting surface 14b. The IOL 40 is placed on the setting surfaces 14a and 14b while the setting part 12 (i.e., the fixed part 12a and the movable part 12b) is opened. In this state, the IOL 40 is not folded (see Fig. 4A). Thereafter, when the setting part 12 (i.e., the fixed part 12a and the movable part 12b) is closed, the shape (in section) of the setting surfaces 14a and 14b becomes substantially equal to the shape of a semicircular opening 11b of the insertion part 11 opening into the setting part 12 (see Fig. 4B). Thus, the IOL 40 placed on the setting surfaces 14a and 14b is correspondingly folded along the shape of the setting surfaces 14a and 14b. To be more specific, the setting surfaces 14a and 14b have a curve (a substantially semicircular section) that allows the IOL 40 to be held in an unfolded state while the setting part 12 is opened and in a folded state when the setting part 12 is closed.

As shown in Fig. 3B, the setting surfaces 14a and 14b are formed to have a shorter length in an insertion direction (corresponding to a longitudinal direction of the injector 1) than a total length of the IOL 40 so that one of the support parts 42, a rear one (a lower one in Fig. 3B), protrudes backward (downward in Fig. 3B) from the setting surfaces 14a and 14b when the IOL 40 (the optical part 41) is placed on the setting surfaces 14a and 14b. Further, the setting part 12 is formed to have a shorter length in the insertion direction than a total length of a mounting part 21 of the main unit 20 as shown in Fig. 1A. This provides a space for allowing a user to grasp the rear support part 42 with forceps or the like for fine adjustment of the position of the IOL 40 after the lens holding unit 10 is mounted in the main unit 20.

The fixed part 12a and the movable part 12b are provided with covers 15a and 15b respectively. These covers 15a and 15b are formed to cover the setting surfaces 14a and 14b above them when the setting part 12 (the fixed part 12a and the movable part 12b) is closed. The cover 15b is provided at an end facing the cover 15a with a protrusion 16 extending downward (in Fig. 4B) toward the setting surfaces 14a and 14b. This protrusion 16 serves to restrict improper folding of the IOL 40 in a direction other than an opening and closing direction of the setting part 12 corresponding to a folding direction of the IOL 40.

Slanting surfaces 17a and 17b (applied with oblique lines in the figures for convenience of explanation) are formed to make it easy for the user to insert the IOL 40 in the lens holding unit 10 from the back and placed the IOL 40 on the setting surfaces 14a and 14b. A lug 18 formed of a flat plate is provided on the side of the fixed part 12a. This lug 18 is used as a grip portion to be held by the user with his hand (fingers) to allow the user to mount the lens holding unit 10 in the main unit 20. Although the lug 18 in the present embodiment is provided in the fixed part 12a, it may be provided in the movable part 12b.

The IOL 40 is placed in the lens holding unit 10 with the setting part 12 opened as shown in Fig. 4A. When the lens holding unit 10 is mounted in the main unit 20, the setting part 12 is closed as shown in Fig. 4B, causing folding of the IOL 40. A commercially available foldable intraocular lens may be used as the IOL 40.

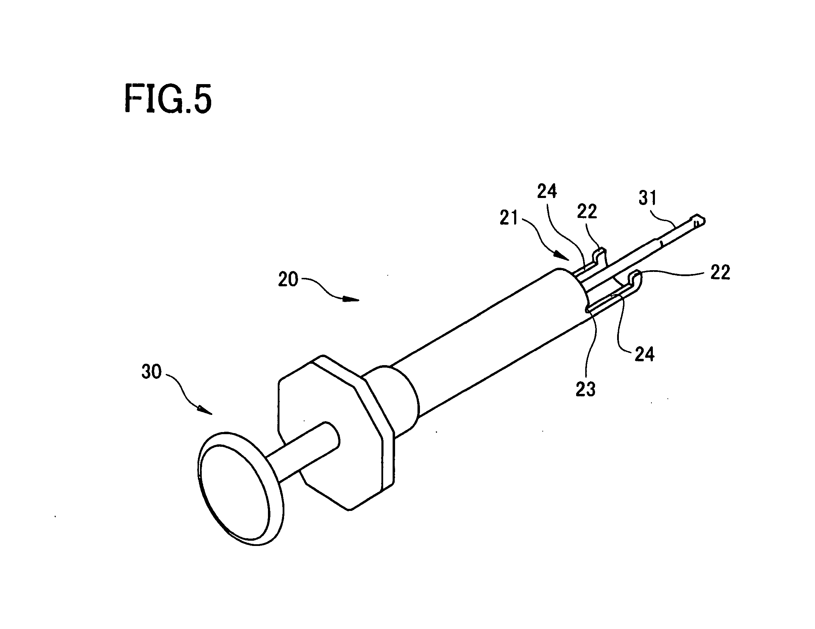

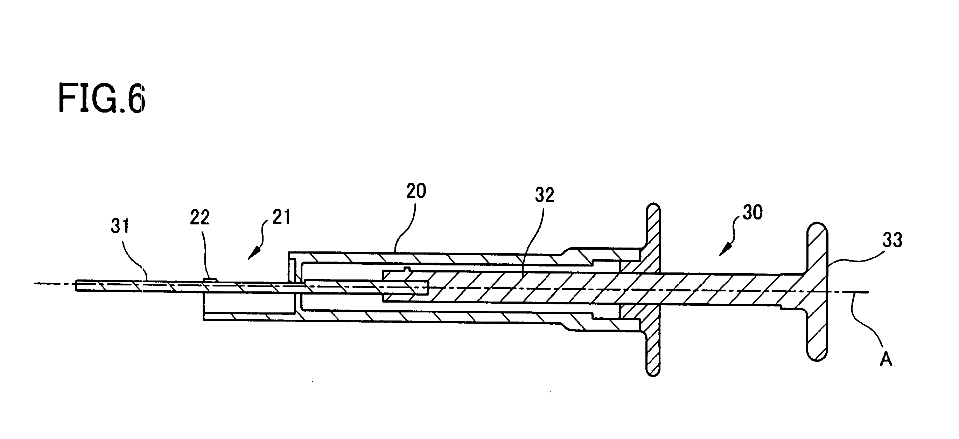

Figs. 5 and 6 are schematic structural views of the main unit 20 and the push unit 30; Fig. 5 is a perspective view of them seen from behind and Fig. 6 is a sectional view of them. The main unit 20 is provided in the forward part (on the tip end side) with a mounting part 21 in which the lens holding unit 10 is to be mounted. This mounting part 21 is of a substantially half shape (in diametrical section) of the main unit 20 and is formed, in the front, with protrusions 22 in symmetrical relation with respect to a central axis A (see Fig. 6) of the main unit 20. The protrusions 22 are positioned slightly above the central axis A and have a distance therebetween slightly narrower (shorter) than the inner diameter of the main unit 20. With this structure, the protrusions 22 serve to guide the setting part 12 in a closing motion when the lens holding unit 10 is mounted in the mounting part 21. Further, the protrusions 22 engage the lens holding unit 10 to prevent the unit 10 from easily becoming detached from the mounting part 21. As with the protrusions 22, both edges 24 of the mounting part 21 also serve to guide the setting part 12 in the closing motion.

The mounting part 21 is formed in the left back (a rear end of the edge 24, i.e., a right end in Fig. 1B) with a recess 23. This recess 23 serves to guide the setting part 12 in the closing motion when the lens holding unit 10 is mounted in the mounting part 21. Further, the recess 23 receives part of a rear edge of the lug 18 (see Fig. 1B) in engagement relation to prevent the lens holding unit 10 from easily becoming detached. In the present embodiment, the mounting part 21 is also formed in the right back with a recess 23 (see Fig. 5). Accordingly, a lens holding unit 10 provided with the fixed part 12a and the movable part 12b arranged in counterchanged positions (the lug 18 is correspondingly formed on an opposite side) can be used.

The push unit 30 is inserted movably along the central axis A of the main unit 20. This push unit 30 includes a push portion (push rod) 31, a shaft portion (plunger) 32, and a push member (thumb rest) 33. The push portion 31 is attached to the shaft portion 32 in the front (on the tip end side). The push member 33 is pushed by a finger of the user to move the shaft portion 32 frontward, pushing the IOL 40 frontward and out of the lens holding unit 10 mounted in the mounting part 21.

An operation for mounting the lens holding unit 10 in the mounting part 21 of the injector 1 constructed as above is explained below.

A user holds the lens holding unit 10 by grasping the lug 18 with one hand while picking the IOL 40 with forceps in the other hand into the lens holding unit 10 and place the IOL 40 in the setting part 12 (i.e., on the setting surfaces 14a and 14b). In this state where the lens holding unit 10 is not subjected to stress, the setting part 12 (the fixed part 12a and the movable part 12b) are in an opened state and the IOL 40 placed thereon is held in an unfolded state, that is, the IOL 40 is not subjected to stress.

To mount the lens holding unit 10 in the mounting part 21, thereafter, the user puts the rear end of the lug 18 into engagement with the recess 23 and pushes down the setting part 12 by pressing the bottom against the protrusions 22. Accordingly, the fixed part 12a and the movable part 12b are guided to close. When the user further slides down the setting part 12 into the mounting part 21, the fixed part 12a and the movable part 12b are fully closed and mounted in the mounting part 21, as shown Fig. 4B.

In the closed state of the setting part 12 (the fixed part 12a and the movable part 12b), the width (distance) of the setting surfaces 14a and 14b in diametrical section is narrower. At this time, the IOL 40 placed on the setting surfaces 14a and 14b is subjected to stress and correspondingly folded along the curved setting surfaces 14a and 14b.

The mounting part of the present invention is required only to have a structure that does not apply stress to the IOL 40 to hold the IOL 40 in an unfolded state before the lens holding unit 10 is mounted in the mounting part, whereas applies stress to the IOL 40 to fold and place the IOL 40 when the lens holding unit 10 is mounted in the mounting part.

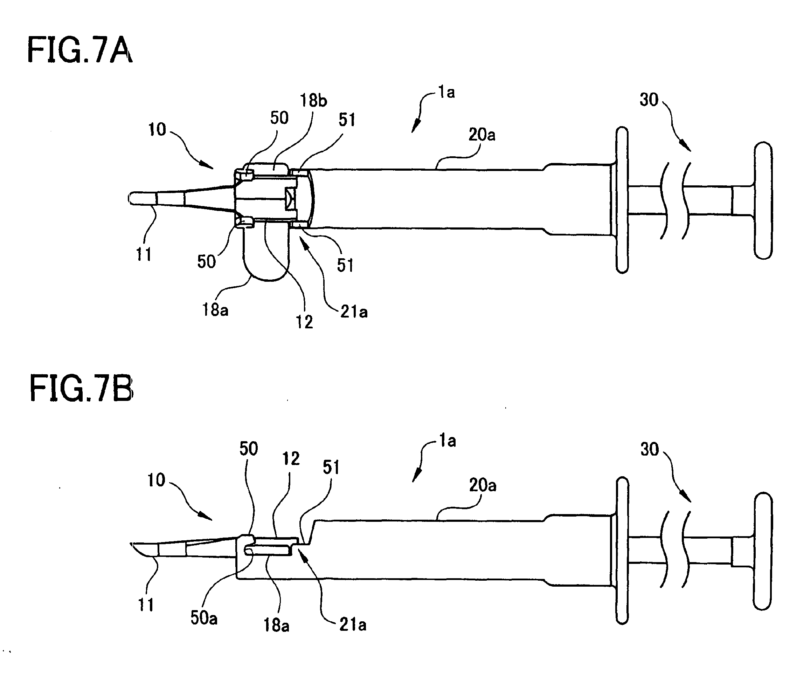

Figs. 7A and 7B are schematic structural views of a second embodiment of the injector according to the present invention. Specifically, Fig. 7A is a top view of an injector 1a and Fig. 7B is a side view of the same. Elements having the same functions as those in the first embodiment described referring to Figs. 1 through 6 are indicated with the same reference numerals and their explanations are omitted.

As shown in Figs. 7A and 7B, a mounting part 21a is formed in the forward part (on the tip end side) with first protrusions 50 positioned in symmetrical relation with respect to a central axis A of a main unit 20a. These first protrusions 50 engage the lens holding unit 10 mounted in the mounting part 21a to prevent the lens holding unit 10 from becoming detached (or coming off) and to guide the setting part 12 in a closing motion. The mounting part 21a is also formed at the back (opposite the tip end) with second protrusions 51 positioned in symmetrical relation with respect to the central axis A. These second protrusions 51 engage the lens holding unit 10 to prevent the lent holding unit 10 from becoming detached (or coming off) and serve to guide the setting part 12 in the closing motion. The first and second protrusions 50 and 51 are positioned slightly above the central axis A. A distance between the first protrusions 50 and a distance between the second protrusions 51 are slightly narrower (shorter) than the inner diameter of the main unit 20a. Under a left one (a lower one in Fig. 7A) of the first protrusions 50, a recess 50a is formed. This recess 50a serves to guide the setting part 12 in the closing motion while it receives part of a front edge of a lug 18a (see Fig. 7B) in engagement relation to prevent the lens holding unit 10 from easily becoming detached. In the present embodiment, another recess 50a is also formed under a right one (an upper one in Fig. 7A) of the first protrusions 50. Accordingly, the lens holding unit 10 provided with the fixed part 12a and the movable part 12b arranged in counterchanged positions (the lug 18 is correspondingly formed on an opposite side) can be used.

It is to be noted that the lug 18 shown in Figs. 1A and 1B is provided close to the back end (the right end in the figures) of the setting part 12 so that the lug 18 is engaged in the recess 23 formed in the rear part of the mounting part 21. On the other hand, the lug 18a shown in Figs. 7A and 7B is provided close to the front end (the left end in the figures) of the setting part 12 so that the lug 18a is engaged in the recess 50a formed in the front part of the mounting part 21a. As shown in Fig. 7A, a small lug 18b may be provided on the right side of the setting part 12 (opposite the lug 18a), so that the small lug 18b is engaged in the recess 50a formed on the right side (on the upper side in Fig. 7A).

To mount the lens holding unit 10 in the mounting part 21a, a user puts the front end of the lug 18a into the recess 50a of the first protrusion 50 and pushes down the setting part 12 by pressing the bottom against the second protrusions 51. Accordingly, the fixed part 12a and the movable part 12b are guided into a closed state. When the user further slides down the setting part 12 into the mounting part 21a, the fixed part 12a and the movable part 12b are fully closed and mounted in the mounting part 21a.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An intraocular lens injector (1, 1a) for injecting a foldable intraocular lens (10) in an eye, including:
a lens holding unit (10) including a cylindrical insertion part (11) having a tip end (11a) which is insertable through an incision made in an eyeball and a setting part (12) disposed opposite the tip end of the insertion part, in which the intraocular lens is placeable, the setting part being openable and closable in a direction of folding the placed intraocular lens;
a cylindrical main unit (20, 20a) including a mounting part (21, 21a) in which the lens holding unit holding the intraocular lens in a folded state in the setting part is mounted; and
a push unit (30) movably disposed in the main unit to push out the intraocular lens placed in the setting part to outside through the tip end of the insertion part;
**characterized in that**
the setting part (12) includes a fixed part (12a) fixed in the insertion part and a movable part (12b) connected with the fixed part with a hinge (13) about which the setting part (12) being openable and closable to fold the intraocular lens,
the mounting part (21, 21a) has guide and engagement means (22, 23, 24, 50, 50a, 51) which applies stress in a direction of closing the fixed part (12a) and the movable part (12b) when the lens holding unit (10) is mounted in the mounting part (21, 21a) and which engages the lens holding unit (10) to prevent the lens holding unit (10) from becoming detached from the mounting part (21, 21a).

2. The intraocular lens injector according to claim 1, wherein the setting part (12) includes a grip portion (18, 18a) which is held by a user to mount the lens holding unit in the mounting part.

3. The intraocular lens injector according to claim 2, wherein the guide and engagement means includes a recess (23, 50a) in which at least one of the setting part and the grip portion is engaged.

4. The intraocular lens injector according to any one of claims 1-3, wherein the setting part (12) includes a setting surface (14a, 14b) having a curve so that the intraocular lens placed in the setting part is held in an unfolded state while the setting part is opened, whereas the lens placed in the setting part is held in a folded state when the setting part is closed.

5. The intraocular lens injector according to claim 4, wherein
the setting surface (14a, 14b) is formed to have a shorter length in an insertion direction than a total length of the intraocular lens, and
the setting part (12) is formed to have a shorter length in the insertion direction than a total length of the mounting part.

6. The intraocular lens injector according to any one of claims 1-5, wherein the setting part (12) includes restricting means (16) for restricting folding of the intraocular lens in a direction other than the predetermined folding direction.

## Patentansprüche

1. Intraokularlinseneinsetzvorrichtung (1, 1a) zum Einsetzen einer faltbaren Intraokularlinse in ein Auge, mit
einer Linsenhalteeinheit (10), die einen zylindrischen Einsetzabschnitt (11) mit einem Spitzenende (11a), das durch einen in einem Augapfel durchgeführten Einschnitt einsetzbar ist, und einen Ladeabschnitt (12), der dem Spitzenende des Einsetzabschnitts gegenüber angeordnet ist und in den die Intraokularlinse einsetzbar ist, enthält, wobei der Ladeabschnitt in einer Richtung des Faltens der eingelegten Intraokularlinse geöffnet und geschlossen werden kann,
einer zylindrischen Haupteinheit (20, 20a), die einen Montageabschnitt (21, 21a) enthält, in dem die Linsenhalteeinheit, die die Intraokularlinse in einem gefalteten Zustand in dem Ladeabschnitt hält, angebracht ist, und
einer Drückeinheit (30), die beweglich in der Haupteinheit angeordnet ist, zum Hinausdrücken der in den Ladeabschnitt eingelegten Intraokularlinse über das Spitzenende des Einsetzabschnitts nach draußen;
**dadurch gekennzeichnet, dass**
der Ladeabschnitt (12) einen festen Abschnitt (12a), der an dem Einsetzabschnitt (11) befestigt ist, und einen beweglichen Abschnitt (12b) enthält, der mit dem festen Abschnitt (12a) über ein Scharnier (13) verbunden ist, um das der Ladeabschnitt (12)geöffnet und geschlossen werden kann, um die Intraokularlinse zu falten,
der Montageabschnitt (21, 21a) ein Führungs- und Eingriffmittel (22, 23, 24; 50, 50a, 51) enthält, das eine Belastung in einer Richtung des Schließens des festen Abschnitts (12a) und des beweglichen Abschnitts (12b) aufbringt, wenn die Linsenhalteeinheit (10) in dem Montageabschnitt (21, 21a) angebracht wird, um zu verhindern, dass die Linsenhalteeinheit aus dem Montageabschnitt (21, 21a) gelöst wird.

2. Intraokularlinseneinsetzvorrichtung nach Anspruch 1, bei der der Ladeabschnitt (12) einen Griffabschnitt (18, 18a) enthält, der von einem Benutzer gehalten wird, um die Linsenhalteeinheit in dem Montageabschnitt anzubringen.

3. Intraokularlinseneinsetzvorrichtung nach Anspruch 2, bei der das Führungs- und Eingriffmittel eine Ausnehmung (23, 50a) enthält, in die zumindest der Ladeabschnitt oder der Griffabschnitt eingreift.

4. Intraokularlinseneinsetzvorrichtung nach einem der Ansprüche 1 bis 3, bei der der Ladeabschnitt (12) eine Ladefläche (14a, 14b) mit einer Kurve enthält, so dass die in den Ladeabschnitt eingelegte Linse in einem ungefalteten Zustand gehalten wird, wenn der Ladeabschnitt geöffnet ist, wohingegen die in den Ladeabschnitt eingelegte Linse in einem gefalteten Zustand gehalten wird, wenn der Ladeabschnitt geschlossen ist.

5. Intraokularlinseneinsetzvorrichtung nach Anspruch 4, bei der
die Ladefläche (14a, 14b) so ausgebildet ist, dass sie in einer Einsetzrichtung eine kürzere Länge hat als eine Gesamtlänge der intraokularen Linse, und
der Ladeabschnitt (12) so ausgebildet ist, dass er in der Einsetzrichtung eine kürzere Länge hat als eine Gesamtlänge des Montageabschnitts.

6. Intraokularlinseneinsetzvorrichtung nach einem der Ansprüche 1 bis 5, bei der der Ladeabschnitt (12) ein Einschränkungsmittel (16) aufweist zum Einschränken des Faltens der Intraokularlinse in einer anderen Richtung als der vorbestimmten Faltrichtung

## Revendications

1. Injecteur de lentille intraoculaire (1, 1a) pour l'injection d'une lentille intraoculaire pliable (40) dans un oeil, comprenant:
une unité de maintien d'une lentille (10) comprenant une partie cylindrique d'insertion (11), elle-même munie d'une extrémité pointue (11a) qui peut être introduite par l'intermédiaire d'une incision faite dans un globe oculaire, et une partie de fixation (12) située à l'opposé de l'extrémité pointue de la partie d'insertion dans laquelle on place la lentille intraoculaire, la partie de fixation s'ouvrant et se fermant dans la direction de pliage de la lentille intraoculaire mise en place ;
une unité principale cylindrique (20, 20a) comprenant une partie de montage (21, 21a) dans laquelle se trouve l'unité de maintien de la lentille qui maintient la lentille intraoculaire pliée dans la partie de fixation ; et
une unité de poussée (30) disposée de façon à se déplacer dans l'unité principale pour pousser la lentille intraoculaire placée dans la partie de fixation hors de l'extrémité pointue de la partie d'insertion ;
**caractérisé en ce que**
la partie de fixation (12) comprend une partie fixe (12a) fixée dans la partie d'insertion et une partie mobile (12b) reliée à la partie fixe par une charnière (13) permettant l'ouverture et la fermeture de la partie de fixation (12) pour le pliage de la lentille intraoculaire ,
la partie de montage (21, 21a) dispose de moyens de guidage et d'engagement (22, 24, 50, 51) qui appliquent une contrainte dans la direction de fermeture de la partie fixe (12a) et de la partie mobile (12b) quand l'unité de maintien de la lentille (10) est montée dans la partie de montage (21, 21a), ce qui engage l'unité de maintien de la lentille (10) et empêche cette unité (10) de se séparer de la partie de montage (21, 21a).

2. Injecteur de lentille intraoculaire selon la revendication 1, dans lequel la partie de fixation (12) comprend une partie préhensible (18, 18a) que l'utilisateur saisit pour installer l'unité de maintien de la lentille dans la partie de montage.

3. Injecteur de lentille intraoculaire selon la revendication 2, dans lequel les moyens de guidage et d'engagement comprennent une encoche (23, 50a) dans laquelle au moins une des parties de fixation et de préhension est engagée.

4. Injecteur de lentille intraoculaire selon l'une quelconque des revendications 1 à 3, dans lequel la partie de fixation (12) comprend une surface de fixation (14a, 14b) incurvée telle que, lorsque la partie de fixation est ouverte, la lentille intraoculaire se trouvant dans la partie de fixation est dépliée, et lorsque la partie de fixation est fermée, la lentille intraoculaire se trouvant dans la partie de fixation est pliée.

5. Injecteur de lentille intraoculaire selon la revendication 4 dans lequel
la surface de fixation (14a, 14b) est réalisée de manière à ce que sa longueur soit plus courte que la longueur totale de la lentille intraoculaire dans la direction d'insertion, et
la partie de fixation (12) est réalisée de manière à ce que sa longueur soit plus courte que la longueur totale de la partie de montage dans la direction d'insertion.

6. Injecteur de lentille intraoculaire selon l'une quelconque des revendications 1 à 5, dans lequel la partie de fixation (12) comprend un moyen de restriction (16) limitant le pliage de la lentille intraoculaire dans une direction autre que la direction de pliage prédéterminée.
